# EUROPEAN PATENT APPLICATION

(11) **EP 4 455 302 A1**
(43) Date of publication of application: **30.10.2024**
(21) Application number: 23169789.7
(22) Date of filing: 25.04.2023
(51) Int. Cl.: C12Q 1/6839

(54) **PROGRAMMABLE NUCLEASE RESISTANCE OF NUCLEIC ACID ASSEMBLIES**

(71) Applicant: Technische Universität München, 80333 München (DE)
(72) Inventor: DIETZ, Hendrik, Haar (DE)
(74) Representative: Potter Clarkson

(57) **Abstract**

The present invention provides nuclease-resistant nucleic acid nanostructures, pharmaceutical compositions thereof, pharmaceutical and diagnostic uses thereof as well as a method of producing nucleic acid nanostructures.

## Description

### Background of the Invention

DNA nanotechnology is considered to have special potential for the generation of novel products with potential medical or even chemical/physical applications. One problem for an application of DNA nanostructures is the environmental conditions in which such objects retain their form and function. Organisms typically contain DNA-degrading enzymes, so-called nucleases, which can degrade DNA nanostructures.

One solution presented in the past is based on coating nucleic acid nanostructures with polymers such as poly-ethylene glycol (PEG).

Nandhini Ponnuswamy et al., 2017, Nature Communications volume 8, Article number: 15654 describe an oligolysine-based coating which protects DNA nanostructures from low-salt denaturation and nuclease degradation.

WO 2020/210468 A1 describes nucleic acid nanostructures crosslinked with oligolysines.

Frances M. Anastassacos et al., 2020, J. Am. Chem. Soc. 2020, 142, 7, 3311-3315 describe that glutaraldehyde cross-linking of oligolysines coating DNA origami greatly reduces susceptibility to nuclease degradation.

Nayan P. Agarwal et al., 2017, Angewandte Chemie 56, 20, 5460-5464 describe block copolymer micellization as a protection strategy for DNA origami.

The aforementioned methods have substantial limitations in terms of their controllability. Oligolysine-PEG copolymers are coated statistically on the entire nanostructure rather than spatially resolved. Thus, targeted incorporation of weak points, e.g., for programmable release of drugs through a temporal sequence of nuclease degradation-induced conformational changes, is not possible. The PEG shell is stabilized by electrostatic interactions between positively charged oligolysine and negatively charged DNA backbone. These are non-covalent interactions that are potentially subject to dynamic fluctuations. Oligolysine is a peptide and could potentially act immunogenically. PEG could also be immunogenic.

### Object

Therefore, there is still a need for ways to specifically stabilize nucleic acid nanostructures against degradation by nucleases.

### Summary of the Invention

The present invention relates to a nucleic acid nanostructure, preferably a deoxyribonucleic acid (DNA) nanostructure, comprising one or more self-assembling nucleic acid building block(s), preferably DNA building block(s), wherein the nucleic acid nanostructure comprises DNA molecules comprising nuclease-resistant nucleotides on at least a part of a surface of the nucleic acid nanostructure.

The present invention further relates to a pharmaceutical composition comprising the nucleic acid nanostructure as described herein and optionally a pharmaceuticals acceptable excipient.

The present invention further relates to a method of producing a nucleic acid nanostructure, preferably the nucleic acid nanostructure as described herein, the method comprising:
(a) providing one or more self-assembling nucleic acid building block(s), preferably DNA building block(s), and providing DNA molecules comprising nuclease-resistant nucleotides,
   wherein the one or more self-assembling nucleic acid building block(s) comprise one or more oligonucleotide(s) at pre-defined sites of the self-assembling nucleic acid building block(s), preferably at pre-defined sites on a surface of the nucleic acid nanostructure, wherein the one or more oligonucleotide(s) comprise a nucleic acid sequence stretch which is complementary to one or more DNA sequence stretch(es) of the DNA molecules comprising nuclease-resistant nucleotides; and
(b) subjecting the one or more self-assembling nucleic acid building block(s) and the DNA molecules comprising nuclease-resistant nucleotides to conditions which allow complementary base pairing.

### Brief Description of the Figures

**Figure 1**: (from Castro et al., Primer to scaffolded DNA origami, Nature Methods 2010). Schematic representation of DNA nanostructures constructed with DNA origami. Thin lines represent DNA strands. Cylinders represent DNA double helices. Structures are constructed by hybridizing sequence-complementary DNA molecules to form cross-linked strand backbone junctions.
**Figure 2**: Programmable nuclease resistance of DNA assemblies, schematic representation of three exemplary embodiments.
**Figure 3**: Protective coat solutions with designed weak sites. By omitting or thinning the nuclease-resistant layer, desired sites can be selectively exposed to accelerated nuclease degradation.
**Figure 4**: Schematic representation of the structure of protective oligonucleotides. Each unit can in principle be attached to a carrier structure (e.g., a DNA origami) at desired positions via base complementarity.
**Figure 5**: CadnanoSQ file of the s16hb with 25 single-stranded handles (left); cadnanoSQ file of the b36hb with 98 single-stranded handles (right).
**Figure 6A**: Schematic illustration of a nuclease degradation protection assay; **B**: DNase I degradation analysis of s16hb. s16hb with DNA handle coating (left) and with LNA handle coating (right).
**Figure 7A**: Gel electrophoretic mobility of b36hb. 55 % Fetal Bovine Serum (FBS) was added to DNA coated b36hb (left) and LNA coated b36hb (right); **B**: Negative stain TEM characterization of the degradation of b36hb in 55 % FBS. All scale bars: 100 nm.
**Figure 8**: Length measurement of the fully coated b36hb throughout 24 h of the DNase I degradation.

### Detailed Description of the Invention

Unless defined otherwise, all technical and scientific terms used herein have the same meaning as commonly understood by those of ordinary skill in the art to which this invention pertains.

The term "comprising" or "comprises" as used herein means "including, but not limited to". The term is intended to be open-ended, to specify the presence of any stated features, elements, integers, steps, or components, but not to preclude the presence or addition of one or more other features, elements, integers, steps, components, or groups thereof. The term "comprising" or "comprises" thus includes the more restrictive terms "consisting of" and "consisting essentially of". In one embodiment, the term "comprising" or comprises" as used throughout the application and in particular within the embodiments may be replaced by the term "consisting of" or "consisting essentially of".

The terms "a" and "an" and "the" and similar references in the context of describing the invention (especially in the context of the following embodiments) are to be construed to cover both the singular and the plural, unless otherwise indicated herein or clearly contradicted by context. For example, the term "a DNA molecule" includes a plurality of DNA molecules, including mixtures thereof. Where the plural form is used for compounds, salts, and the like, this is taken to mean also a single compound, salt, or the like.

In the context of the present disclosure, the term "DNA" refers to deoxyribonucleic acid composed of a single-strand of monomeric units called nucleotides, wherein each nucleotide is composed of a nitrogen-containing nucleobase, a 2-deoxyribose sugar moiety, and a phosphate group, wherein the individual nucleotides are linked in the single-strand by a phosphate group linking the OH group in position 5' of a 2-deoxyribose sugar moiety to the OH group in 3' of a neighboring 2-deoxyribose sugar moiety. In particular embodiments, the nitrogen-containing nucleobases are independently selected from cytosine [C], guanine [G], adenine [A] and thymine [T] In particular embodiments, one or more of the nucleobases are non-canonical bases, in particular a non-canonical base selected from the list of: a modified adenosine, in particular N6-carbamoyl-methyladenine or N6-methyadenine; a modified guanine, in particular 7-deazaguanine or 7- methylguanine; a modified cytosine, N4-methylcytosine, 5-carboxylcytosine, 5-formylcytosine, 5-glycosylhydroxymethylcytosine, 5-hydroxycytosine, or 5- methylcytosine; a modified thymidine, in particular a-glutamyl thymidine or a- putrescinyl thymine; a uracil or a modification thereof, in particular uracil, base J, 5- dihydroxypentauracil; or 5-hydroxymethyldeoxyuracil; deoxyarchaeosine and 2,6- diaminopurine. A stretch or part of a single strand of DNA may interact with a complementary stretch of DNA by interaction of complementary nucleobases to form a duplex, wherein cytosine and guanine, and adenine and thymine, are complementary to each other, respectively, by forming two (A/T) and three (G/C) hydrogen bonds between the nucleobases. A duplex may be formed by two single-strands of DNA that are fully complementary to each other, as in the case of genomic DNA, or by single-strands of DNA that are partially complementary to each other, including situations, where one single-strand of DNA is partially complementary to two or more other single-stranded DNA strands. A duplex may also be formed by two fully or partially self-complementary stretches of one single stranded of DNA resulting in the formation of a hairpin, a loop or a hybridization stem. In a particular embodiment, the invention provides a nucleic acid nanostructure, which is a DNA or DNA-based nanostructure.

In the context of the present disclosure, the term "RNA" refers to ribonucleic acid composed of a single-strand of monomeric units called nucleotides, wherein each nucleotide is composed of a nitrogen-containing nucleobase, a ribose sugar moiety, and a phosphate group, wherein the individual nucleotides are linked in the single strand by a phosphate group linking the OH group in position 5' of a ribose sugar moiety to the OH group in 3' of a neighboring ribose sugar moiety. In particular embodiments, the nitrogen-containing nucleobases are independently selected from cytosine [C], guanine [G], adenine [A] and uracil [U]. In particular embodiments, one or more of the nucleobases are non-canonical bases, in particular a non-canonical base selected from the list of: pseudouridine, ribothymidine, and inosine. Unlike DNA, RNA is most often in a single-stranded form, but the formation of double-stranded forms is possible by interaction of complementary nucleobases, wherein cytosine and guanine, and adenine and uracil, are complementary to each other, respectively by forming two (A/T) and three (G/C) hydrogen bonds between the nucleobases. In a particular embodiment, the invention provides a nucleic acid nanostructure, which is a RNA or a RNA-based nanostructure.

The present invention describes a programmable method for stabilizing nucleic acid nanostructures, especially also multi-layer DNA origami, against degradation by nucleases. For example, the structures to be protected might be coated in a targeted and spatially resolved manner with nuclease-resistant DNA molecules.

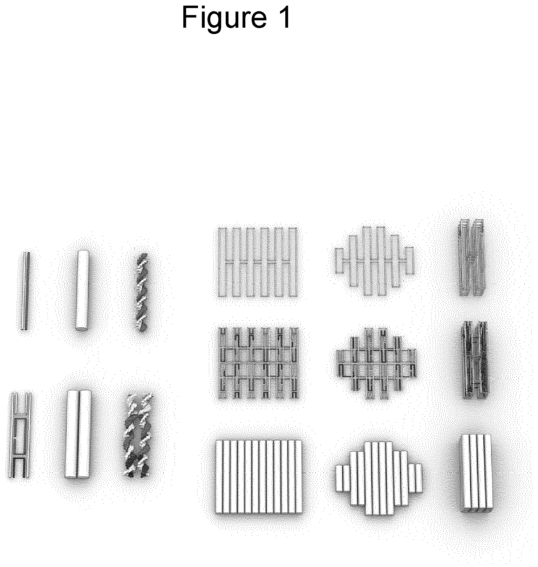

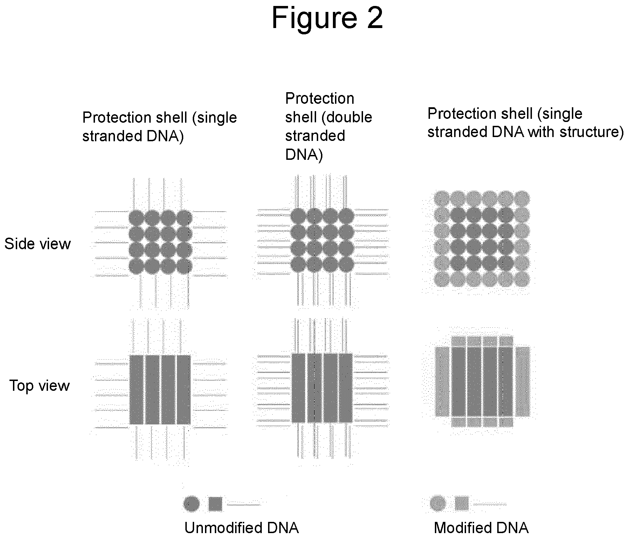

The underlying concept is outlined in more detail in Figures 1 and 2.

In one aspect, the present invention relates to a nucleic acid nanostructure, preferably a DNA nanostructure, comprising one or more self-assembling nucleic acid building blocks, preferably DNA building blocks, wherein the nucleic acid nanostructure comprises DNA molecules comprising nuclease-resistant nucleotides on at least a part of a surface of the nucleic acid nanostructure.

A "nucleic acid nanostructure," as used herein, refers to nucleic acids that form (e.g., self-assemble) two-dimensional (2D) or three-dimensional (3D) shapes (e.g., reviewed in W.M. Shih, C. Lin, Curr. Opin. Struct. Biol.20, 276 (2010), incorporated by reference herein). Nanostructures may be formed using any nucleic acid folding or hybridization methodology. One such methodology is DNA origami (see, e.g., Rothmund, P.W.K. Nature 440 (7082): 297-302 (2006), incorporated by reference herein). In a DNA origami approach, a nanostructure is produced by the folding of a longer "scaffold" or "template" nucleic acid strand through its hybridization to a plurality of shorter "staple" oligonucleotides, each of which hybridize to two or more non-contiguous regions within the scaffold strand. In some embodiments, a scaffold strand is at least 100 nucleotides in length. In some embodiments, a scaffold strand is at least 500, at least 1000, at least 2000, at least 3000, at least 4000, at least 5000, at least 6000, at least 7000, or at least 8000 nucleotides in length. The scaffold strand may be naturally or non-naturally occurring. Staple strands are typically less than 100 nucleotides in length; however, they may be longer or shorter depending on the application and depending upon the length of the scaffold strand. In some embodiments, a staple strand may be 15 to 100 nucleotides in length. In some embodiments, a staple strand is 25 to 50 nucleotides in length.

In some embodiments, a nucleic acid nanostructure may be assembled in the absence of a scaffold strand (e.g., a scaffold-free structure). For example, a number of oligonucleotides (e.g., less than 200 nucleotides or less than 100 nucleotides in length) may be assembled to form a nucleic acid nanostructure.

A nucleic acid nanostructure may be assembled into one of many defined and predetermined shapes including without limitation a capsule, hemi-sphere, a cube, a cuboidal, a tetrahedron, a cylinder, a cone, an octahedron, a prism, a sphere, a pyramid, a dodecahedron, a tube, an irregular shape, and an abstract shape. The nanostructure may have a void volume (e.g., it may be partially or wholly hollow). In some embodiments, the void volume may be at least 25 %, at least 50%, at least 75%, at least 85%, at least 90%, or more of the volume of the nanostructure. Thus, in some embodiments, nanostructures do not comprise a solid core. In some embodiments, nanostructures are not circular or near circular in shape. In some embodiments, nanostructures are not a solid core sphere. Depending on the intended use, nanostructures may be assembled into a shape as simple as a two-dimensional sheet or as complex as a three-dimensional capsule or lattice (or even more complex).

In some embodiments, nucleic acid nanostructures are rationally designed. A nucleic acid nanostructure is herein considered to be "rationally designed" if nucleic acids that form the nanostructure are selected based on pre-determined, predictable nucleotide base pairing interactions that direct nucleic acid hybridization. For example, nanostructures may be designed prior to their synthesis, and their size, shape, complexity and modification may be prescribed and controlled using certain select nucleotides (e.g., oligonucleotides) in the synthesis process. The location of each nucleic acid in the structure may be known and provided for before synthesizing a nanostructure of a particular shape. The fundamental principle for designing, for example, self-assembled nanostructures is that sequence complementarity in nucleic acid strands is selected such that, by pairing up complementary segments, the nucleic acid strands self-organize into a predefined nanostructure under appropriate conditions. Thus, in some embodiments, nanostructures are self-assembling. Similarly, complementary sequence stretches of oligonucleotides for attachment or linkage (e.g., those linking the DNA molecules comprising nuclease-resistant DNA molecules, agents or targeting molecules) may be rationally designed to attach specifically to an interior or exterior surface of a nanostructure, in some embodiments, without intercalation or hybridization with nucleic acids forming the body of the nanostructure such as the self-assembling nucleic acid building blocks, preferably DNA building blocks.

Examples of nucleic acid nanostructures include, without limitation, capsules, lattices (E. Winfree, et al. Nature 394, 539 (1998); H. Yan, et al. Science 301, 1882 (2003); H. Yan, et al. Proc. Natl. Acad. of Sci. USA 100, 8103 (2003); D. Liu, et al. J. Am. Chem. Soc.126, 2324 (2004); P.W.K. Rothemund, et al. PLoS Biology 2, 2041 (2004)), ribbons (S.H. Park, et al. Nano Lett.5, 729 (2005); P. Yin, et al. Science 321, 824 (2008)), tubes (H. Yan Science (2003); P. Yin (2008)), finite two- dimensional (2D) and three dimensional (3D) objects with defined shapes (J. Chen, N. C. Seeman, Nature 350, 631 (1991); P. W. K. Rothemund, Nature 440, 297 (2006); Y. He, et al. Nature 452, 198 (2008); Y. Ke, et al. Nano. Lett.9, 2445 (2009); S. M. Douglas, et al. Nature 459, 414 (2009); H. Dietz, et al. Science 325, 725 (2009); E. S. Andersen, et al. Nature 459, 73 (2009); T. Liedl, et al. Nature Nanotech.5, 520 (2010); D. Han, et al. Science 332, 342 (2011)), and macroscopic crystals (J. P. Meng, et al. Nature 461, 74 (2009)). Other nucleic acid nanostructures may be used as provided herein.

In one embodiment, the nucleic acid nanostructure of the invention is a nanostructure capable of entrapping viruses such as the DNA nanostructures as described in EP 22204809.2, incorporated herein by reference, specifically the nucleic acid nanostructure as described on pages 12-86. In one embodiment, the nucleic acid nanostructure is a nanostructure as described in WO 2021/165528, incorporated herein by reference, specifically on pages 21-60.

In some embodiments, the nucleic acid nanostructure of the invention is a nucleic acid (e.g., DNA) origami nanostructure. In some embodiments, the nucleic acid nanostructure is a nucleic acid (e.g., DNA) single-stranded tile (SST) nanostructure.

Nucleic acid nanostructures may comprise, DNA, RNA, modified DNA, modified RNA, PNA, LNA or a combination thereof. Nucleic acid nanostructures may consist of DNA, RNA, modified DNA, modified RNA, PNA, LNA or a combination thereof.

The size of the DNA molecules comprising nuclease-resistant nucleotides may vary and be dependent on the type of linkage to the self-assembling nucleic acid building blocks, preferably DNA building blocks. For example, the DNA molecules might have a length of about 10 to 500 nucleotides, or about 20 to 300 nucleotides, or about 30 to 150 nucleotides, or about 50 to 100 nucleotides. In one embodiment, the DNA molecules might be linked to the nucleic acid building blocks by their terminus, e.g., they protrude from the self-assembling nucleic building blocks. In other embodiments, the DNA molecules comprising nuclease-resistant nucleotides align with the self-assembling nucleic acid building blocks, e.g., by interhelical interactions.

In one embodiment, the DNA molecules comprise nuclease-resistant nucleotides with random bases. The term "random bases" as used herein, refers to a random composition of bases of the nucleotides, preferably A, T, G or C, a random sequence of the individual bases and/or a random nature or bases, e.g., non-canonical bases as described herein. Basically, the individual bases should not interfere with the base pairing and/or phosphodiester bonding properties between single nucleotides.

In one embodiment, the DNA molecules comprising nuclease-resistant nucleotides are not branched molecules.

In one embodiment, the DNA molecule comprising nuclease-resistant nucleotides are linear molecules.

The size and nature of the surface of the nucleic acid nanostructure may depend on the specific 3- or 2-dimensional structure of the nucleic acid nanostructure. The term refers to the exterior surface as well as to the interior surface of a nucleic acid nanostructure. For example, in case the nucleic acid nanostructure is a shell, the inside surface of the shell is also comprised by the term surface.

In one embodiment, the nucleic acid nanostructure comprises DNA molecules comprising nuclease-resistant nucleotides on at least a part of an exterior surface of the nanostructure.

In one embodiment, the nucleic acid nanostructure comprises DNA molecules comprising nuclease-resistant nucleotides on at least a part of an interior surface of the nanostructure.

In one embodiment, the nucleic acid nanostructure comprises DNA molecules comprising nuclease-resistant nucleotides on the complete surface of the nanostructure.

Nucleic acids such as cellular DNA and RNA, synthetic oligonucleotides are prone to degradation once introduced into a cell or body fluids. Such degradation is due to the ubiquitous presence of nuclease enzymes (both exonucleases and endonucleases), as well as chemical instability (particularly for RNA). Under normal cellular conditions, this leads to fast *in vivo* degradation and a short half-life. "Nuclease-resistance" as used herein refers to a resistance of DNA molecules which comprise nuclease-resistant nucleotides which is increased compared to DNA molecules which do not comprise nuclease resistant nucleotides, preferably under physiological conditions. Nuclease resistance might be accomplished by modifications of the nucleotides such as chemical modifications of specific nucleotide moieties.

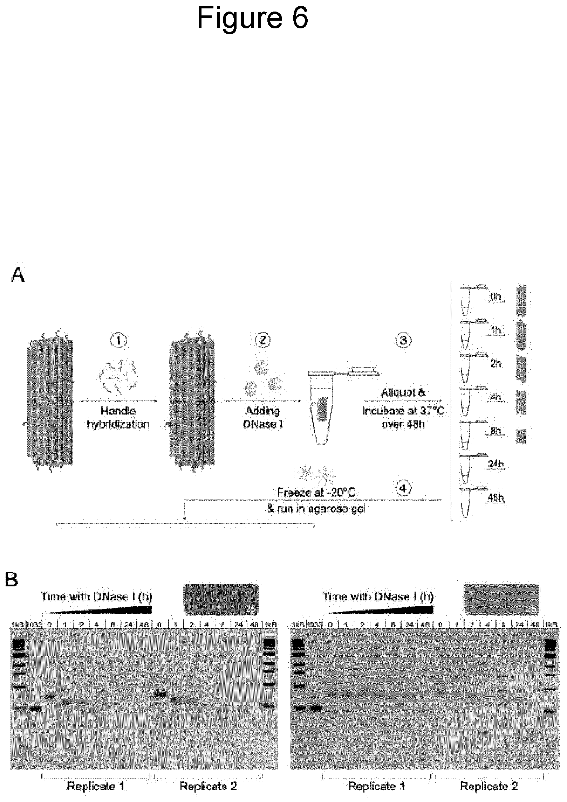

Nuclease-resistance can be measured by any method known in the art including but not limited to incubation of a nucleic acid nanostructure to be tested in 55 % Fetal Bovine Serum (FBS) or in other solutions containing nucleases, in particular DNAse I enzymes, for at least about 1 h, at least about 4 h, at least about 6 h, at least about 8 h, at least about 10 h, at least about 12 h, at least about 24 h, at least about 48 h, at least about 3 days, at least about 4 days, or about 1 to 24 h, preferably 6 to 12 h, more preferably 8 h at about 22 to 22° C. Subsequent analysis of degradation of the nucleic acid nanostructure to be tested, preferably in comparison to a control, is performed by gel electrophoretic mobility analysis and/or electron microscopic analysis such as transmission electron microscopy (TEM), e.g., as shown and illustrated in the Examples and Figures 6 to 8. In one embodiment, the nucleic acid nanostructure to be tested is a nucleic acid nanostructure of the present invention, e.g., a nucleic acid nanostructure, preferably a deoxyribonucleic acid (DNA) nanostructure, comprising one or more self-assembling nucleic acid building block(s), preferably DNA building block(s), wherein the nucleic acid nanostructure comprises DNA molecules comprising nuclease-resistant nucleotides on at least a part of a surface of the nucleic acid nanostructure and the control is a nucleic acid nanostructure with the same self-assembling nucleic acid building block(s) comprising a different nuclease-resistant coating, e.g. a PEG coating or PEG-oligolysine coating. Alternatively, the control might by a nucleic acid nanostructure with the same self-assembling nucleic acid building block(s) as the building blocks of the nucleic acid nanostructure to be tested comprising DNA molecules without nuclease-resistant nucleotides, preferably on the same parts of the surface of the nanostructure as the nucleic acid nanostructure to be tested, or the control might be a nucleic acid nanostructure with the same self-assembling nucleic acid building block(s) as the building blocks of the nucleic acid nanostructure to be tested without a coating or DNA molecules on at least a part of the surface of the nanostructure.

In accordance with the invention, any modification of a nucleotide which leads to a nuclease resistance might be used. In one embodiment, the nuclease-resistant nucleotides comprise a modification in the sugar moiety and/or the phosphate moiety, in particular a bridged sugar moiety such as locked-nucleic acids (LNA) wherein the ribose moiety is modified with an extra bridge connecting the 2' oxygen and 4' carbon, a 2',4'-OMethyl bridge, an 2',4'-OEthyl bridge, an L-sugar moiety, e.g., an L-2'deoxyribose or an L-2'ribose, a peptide nucleic acid, and/or a phosphorothioate.

In one embodiment, the nucleic acid nanostructure unfolds and/or decomposes in a time-controlled manner, preferably wherein unfolding is controlled by spatial distribution of the DNA molecules comprising nuclease-resistant nucleotides on the at least a part of the surface of the nanostructure.

In one embodiment, the nucleic acid nanostructure unfolds and/or decomposes within about 1 hour to 4 days, or about 4 hours to 2 days, or about 10 hours to 1 day, preferably under conditions as described herein for nuclease-resistance measurement.

The nucleic acids for the one or more self-assembling nucleic acid building blocks, preferably DNA building blocks of DNA nanostructures of the present invention can be produced by a phage-mediated method of scalable biotechnological production of single-stranded(ss) DNA for DNA origami which is described in EP3516055B1 which is incorporated herein by reference. In this approach a phagemid is comprised of user defined sequences interleaved with self-cleavable DNAzyme cassettes. After the ssDNA is produced in phage culture at scale, the target ssDNA strands can be digested, separated, and readily used for assembly of DNA origami.

Any DNAzyme can be used to produce ssDNA strands for the self-assembling DNA building blocks of the DNA nanostructures of the present invention including but not limited to DNAzymes that hydrolyze DNA with high speed and sequence specificity as described in EP 23169206 of the same applicant with the title "Novel self-cleaving DNAzymes and selection process", specifically as described on pages 18-21, incorporated herein by reference, such as DNAzymes with a sequence selected from SEQ ID NOs: 1, 2 and 24-47 and/or as described in Gu *et al.,* 2013, incorporated herein by reference, for example, the DNAzymes, named I-R3. A further example of an DNAzyme that can be used for producing ssDNA s is II-R2/3 DNAzyme as described in Qiao Zhang *et al.,* 2022, incorporated herein by reference.

The DNA molecules comprising nuclease-resistant nucleotides might be linked to the self-assembling nucleic acid building blocks, preferably DNA building blocks, during or after production and/or assembly of the self-assembling building blocks. In one embodiment, the DNA molecules comprising nuclease-resistant nucleotides are linked to the self-assembling building blocks after assembly of the self-assembling building blocks. In one embodiment, the DNA molecules comprising nuclease-resistant nucleotides are linked to the self-assembling building blocks during assembly of the self-assembling building blocks.

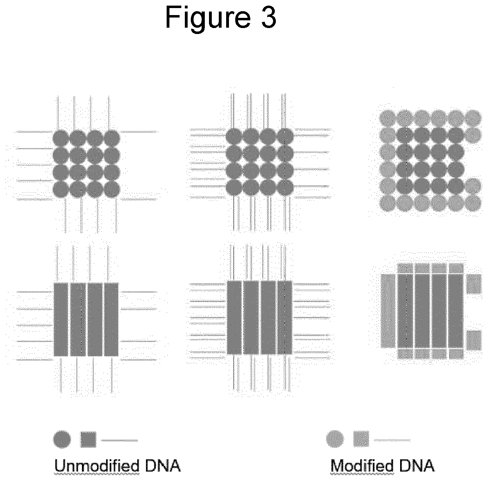

Thus, in one embodiment, the self-assembling nucleic acid building blocks, preferably DNA building blocks, comprise one or more oligonucleotides, wherein the one or more oligonucleotides comprise one or more nucleic acid sequence stretches which are complementary to one or more DNA sequence stretches on the DNA molecules comprising nuclease-resistant nucleotides, wherein preferably the DNA molecules comprising nuclease-resistant nucleotides are linked to the self-assembling building block by complementary interactions. In one embodiment, the DNA molecules comprising nuclease-resistant nucleotides are linked to the self-assembling building block by complementary interactions such as by interaction of complementary nucleobases to form a duplex as described herein. Preferably the one or more oligonucleotides are located at predefined sites of parts of the surface of the nucleic acid nanostructure and/or at a predefined density on the surface of the nucleic acid nanostructure depending on the intended time-controlled unfolding and/or decomposition of the nucleic acid nanostructure. In a specific embodiment, the predefined density is one oligonucleotide per 1 nm² to 15 nm² of the surface of the nucleic acid nanostructure, or one oligonucleotide per 2 nm² to 10 nm² of the surface of the nucleic acid nanostructure, or one oligonucleotide per 4 nm² to 8 nm² of the surface of the nucleic acid nanostructure. Examples of such predefined sites or parts are given in Figure 3.

In one embodiment, the DNA molecules comprising nuclease-resistant nucleotides are covalently linked to the self-assembling building block, including but not limited to ligating modified strand termini via chemical, enzymatic, or photochemical ligation. Such methods are known to the skilled person.

For application in physiological environments, the DNA molecules comprising nuclease-resistant nucleotides are covalently linked to the self-assembling building block by cyclobutane pyrimidine dimers (CPDs) as described in Gerling et al., 2018, Sci. Adv. 4: 1-11 with the title "Sequence programmable covalent bonding of designed DNA assemblies", incorporated herein by reference. Briefly, the nucleic acid nanostructure comprises 2 proximal thymidines, e.g., two thymidines at the terminus of two contiguous ssDNA strands to give rise to half cross over or full cross over bonds or 2 thymidines which are placed in non-duplex loops of a DNA sequence, preferably loops of 5 thymidines in two contiguous DNA sequences to give rise to interhelical bonds and are irradiated with UV light, preferably UV light with about 300-400 nm, more preferably about 310-365 nm to form a cyclobutane thymidine dimer.

The type of linkage may vary depending on the intended use of the nucleic acid nanostructure. For examples, the DNA molecules comprising nuclease-resistant nucleotides might be linked to the self-assembling building block(s) by complementary interaction only. The DNA molecules comprising nuclease-resistant nucleotides might also be linked to the self-assembling building block(s) by covalent interaction only. The DNA molecules comprising nuclease-resistant nucleotides might also be linked to the self-assembling building block(s) by complementary interaction and covalent interaction.

The self-assembling building blocks might be in any 3- or 2- dimensional shape and might vary in accordance with the intended use of the nucleic acid nanostructure as described herein. In one embodiment, the self-assembling building block comprises:
i) a single-stranded DNA template, and
ii) one or more oligonucleotides complementary to said single-stranded DNA template, wherein each of said oligonucleotides is either complementary to one contiguous DNA sequence stretch or to at least two non-contiguous DNA sequence stretches on said single-stranded DNA template.

The length of the DNA sequence stretches may vary and is preferably chosen to provide a linkage between the single stranded DNA template and/or the one or more oligonucleotides which maintains the intended 2D or 3D DNA nanostructure. In one embodiment, the DNA sequence stretches have a length of about 1 to 50 nucleotides, or about 5 to 40 nucleotides, or about 10 to 30 nucleotides, or about 15 to 20 nucleotides.

In one embodiment, the nucleic acid nanostructure is a shell, capsule, sphere, hemi-sphere, cube, cylinder, a spherocylinder, cone, cuboidal, tube, square, lattice, square lattice, a polyhedron, in particular prism, pyramid, a tetrahedron, octahedron, icosahedron, dodecahedron.

In one embodiment, the self-assembling building blocks of the nucleic acid nanostructure can assemble and/or fold into DNA origami structures, (tile-based) DNA nanostructures, or crystalline DNA nanomaterials as described in the prior art, e.g., in WO 2018/054571, incorporated herein by reference, particularly pages 10-14 and the Examples. In one embodiment, the nucleic nanostructure is a structure as described in EP22207474 with the title "DNA Origami Encoding for Mammalian Gene Expression and Cotransfection" which is incorporated herein per reference, particularly pages 5-19.

In a preferred embodiment, the nucleic acid nanostructure is a shell, for example a shell as described herein, preferably with a void volume (e.g., it may be partially or wholly hollow). In some embodiments, the void volume may be at least 25 %, at least 50%, at least 75%, at least 85%, at least 90% or more of the volume of the nanostructure.

In preferred embodiments, the nucleic acid nanostructure is a shell as described in EP 22204809.2, incorporated herein by reference, specifically the nucleic acid nanostructure as described on pages 12-86. In one embodiment, the nucleic acid nanostructure is a nanostructure as described in WO 2012/165528, incorporated herein by reference, specifically on pages 21-60.

Alternatively, the self-assembling building blocks might be assembled as brick with tiles, e.g., by a brick strategy which uses single stranded DNA tiles that connect through complementary domains to form large DNA assemblies in both 2D and 3D structures.

In one embodiment, the DNA molecules comprising the nuclease-resistant nucleotides comprise about 20 % (m/m), or about 30 % (m/m), or about 40 % (m/m), or about 50 % (m/m), or about 60 % (m/m), or about 70 % (m/m), or about 80 % (m/m), or about 90 % (m/m), or about 100 % (m/m) nuclease-resistant nucleotides relative to the total amount of nucleotides of the DNA molecule. In one embodiment, the DNA molecules comprising the nuclease-resistant nucleotides consist of 100 % (m/m) nuclease-resistant nucleotides relative to the total amount of nucleotides of the DNA molecule.

In one embodiment, the DNA molecules comprising the nuclease-resistant nucleotides is a single stranded DNA molecule.

In one embodiment, the DNA molecules comprising the nuclease-resistant nucleotides is a double stranded DNA molecule.

The density of nuclease-resistant nucleotides within a DNA molecule comprising the nuclease-resistant nucleotides may vary depending on the field of use. For example, a specific density of nuclease-resistant nucleotides can be used in each strand of a double stranded DNA molecule to delay nuclease degradation in a user-defined manner, e.g., as shown below in Example 1.

In one embodiment, the DNA molecules comprising the nuclease-resistant nucleotides comprise one or more units comprising the nuclease-resistant nucleotides and one or more DNA units consisting of unmodified nucleotides. Such units comprising the nuclease-resistant nucleotides may have a length of about 1-70 nucleotides, or 5-30 nucleotides or 10-20 nucleotides. In one embodiment, the units comprising the nuclease-resistant nucleotides consist of nuclease-resistant nucleotides. In one embodiment, the one or more units comprising nuclease-resistant nucleotides comprise about 20 % (m/m), or about 30 % (m/m), or about 40 % (m/m), or about 50 % (m/m), or about 60 % (m/m), or about 70 % (m/m), or about 80 % (m/m), or about 90 % (m/m), or about 100 % (m/m) nuclease-resistant nucleotides relative to the total amount of nucleotides of the unit.

As used herein, an "unmodified nucleotide" refers to a nuclease-sensitive nucleotide. For example, the unmodified nucleotide comprises a 2'-deoxyribose sugar moiety, and a phosphate group, preferably wherein individual unmodified nucleotides are linked by a phosphate group linking the OH group in position 5' of a 2-deoxyribose sugar moiety to the OH group in 3' of a neighboring 2-deoxyribose sugar moiety.

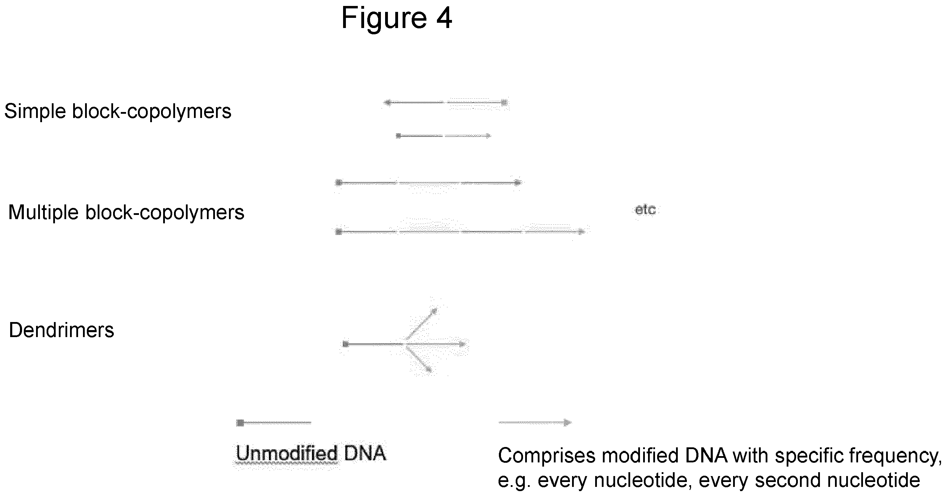

In one embodiment, the one or more units comprising the nuclease-resistant nucleotides and the one or more DNA units consisting of unmodified nucleotides are block copolymers, preferably linear or branched block copolymers or in dendrimers such as block copolymers and dendrimers, e.g., with 2, 3, 4, 5, 6, 7, 8, 9, 10 branches. Further examples are shown in Figure 4.

In one embodiment, the self-assembling building blocks of the nucleic acid nanostructure comprises unmodified nucleotides. In one embodiment, the self-assembling building blocks of the nucleic acid nanostructure consists of unmodified nucleotides, preferably wherein the unmodified nucleotides comprise a 2'-deoxyribose sugar moiety, and a phosphate group, preferably wherein individual unmodified nucleotides are linked by a phosphate group linking the OH group in position 5' of a 2-deoxyribose sugar moiety to the OH group in 3' of a neighboring 2-deoxyribose sugar moiety.

In one embodiment, the nucleic acid nanostructure, in particular the self-assembling nucleic acid building blocks, preferably DNA building blocks, further comprises covalent linkages as described herein, in particular cyclobutane pyrimidine dimers (CPDs). This might lead to a further increased resistance in low ionic strength such as in physiological environment, e.g., a ionic strength of about 150 mM NaCl.

In one embodiment, the nucleic acid nanostructure is linked to an agent such as an active pharmaceutical ingredient or a diagnostic molecule. An agent may be covalently or non-covalently attached to a nucleic acid nanostructure. The location and nature of the linkage between the agent and the nucleic acid nanostructure will depend upon the function of the agent. As an example, an agent may be intended to release (including slow release) from the nucleic acid nanostructure, and in that case, the linkage between the agent and the nanostructure may be chosen to achieve the desired release profile. In some embodiments, an agent may be inactive in its bound form and activated only when released.

In some embodiments, an agent may be combined with nucleic acids during assembly (e.g., self-assembly) of nucleic acid nanostructures, or an agent may be combined with preformed nucleic acid nanostructures. Agents may be linked to an interior surface (in the interior compartment) or an exterior surface of a nanostructure. Agents may be arranged in various configurations. Upon hybridization of complementary nucleic acid sequence stretches of oligonucleotides, agents become indirectly linked to nucleic acid nanostructures. In some embodiments, the exterior surface of a nanostructure contains a and targeting molecules (e.g., antibody fragments such as scFv fragments), and the interior surface of the nucleic acid nanostructure preferably contains a combination of tracking dye and antigen. In some embodiments, the interior surface of a nanostructure contains a and targeting molecules (e.g., antibody fragments such as scFv fragments), and the exterior surface of the nucleic acid nanostructure preferably contains a combination of tracking dye and antigen. Nucleic acid nanostructures of the present disclosure permit precise placement of an agent or more than one agent (e.g., a combination of different agents) on the interior and/or exterior surface of the nanostructures.

The present invention contemplates, in some respects, the delivery of nucleic acid nanostructures or DNA nanostructures loaded with an agent, systemically or to localized regions, tissues or cells. Any agent may be delivered using the methods of the present disclosure provided that it can be loaded onto or into the nanostructure. Because such processes are relatively innocuous, it is expected that virtually any agent may be used.

An agent for use in accordance with the present disclosure may be a protein-based agent (including a protein), a nucleic-acid based agent (including a nucleic acid), a chemical- based agent (including chemical compounds) or combination of any two or more of the foregoing.

Examples of protein-based and peptide-based agents (e.g., therapeutic, prophylactic and/or diagnostic protein-based agents) for use in accordance with the present disclosure include, without limitation, antibodies (e.g., monoclonal antibodies, chimeric antibodies, humanized antibodies), antibody fragments (e.g., single- or multi-chain antibodies, antibody fragments such as F_{ab} fragments, F_{c} fragments), enzymes, co-factors, receptors, ligands, transcription factors and other regulatory factors, antigens, cytokines, chemokines and hormones.

Examples of nucleic acid-based agents (e.g., therapeutic, prophylactic and/or diagnostic nucleic acid-based agents) for use in accordance with the present disclosure include, without limitation, RNA interference molecules such as short-interfering RNA (siRNA) molecules, short-hairpin RNA (shRNA) molecules, and micro-RNA (miRNA) molecules. Nucleic acid-based agents may be recombinant (e.g., non-naturally occurring molecule produced by joining two different nucleic acids) or synthetic (e.g., chemically or otherwise synthesized). In one embodiment, the nucleic nanostructure loaded with a nucleic acid is a structure and an nucleic acid as described in EP22207474 with the title "DNA Origami Encoding for Mammalian Gene Expression and Cotransfection" which is incorporated herein per reference, particularly pages 5-19.

Examples of chemical-based agents (e.g., therapeutic, prophylactic and/or diagnostic chemical-based agents) for use in accordance with the present disclosure include, without limitation, small molecules (e.g., small molecule drugs). A "small molecule" is a low molecular weight (e.g., <900 Daltons) organic compound.

An "active pharmaceutical ingredient" is an agent used to treat a condition in a subject (e.g., human or non-human subject). Examples of active pharmaceutical ingredient for use in accordance with the present disclosure include, without limitation, the agents as described herein, antibodies, antibody fragments, other proteins and peptides, lipids, carbohydrates, small molecules, polymers, metal nanoparticles, RNA interference molecules (e.g., siRNAs, shRNAs, miRNAs), antisense molecules, antigens (e.g., peptide antigens), adjuvants (e.g., CpG oligonucleotides), anti-neoplastic agents, anti-cancer, anti-infective agents (e.g., anti- microbial agents, anti-bacterial agents), anti-fungal agents, anti-viral agents (e.g., anti- retroviral agents), anti-inflammatory agents, metabolic agents, immunomodulatory agents (e.g., immunostimulatory agents, immunosuppressive agents), anti-hypertensive agents, anti- Alzheimer's agents, and anti-Parkinson's agents.

A "diagnostic molecule" is an agent used to diagnose a condition in a subject (e.g., human or non-human subject). Examples of diagnostic molecules for use in accordance with the present disclosure include, without limitation, imaging agents (e.g., contrast agents, radioactive agents, tracking dyes (e.g., fluorescent dyes)). An "imaging agent" is an agent that emits signal directly or indirectly thereby allowing its detection in vivo. Imaging agents, such as contrast agents and radioactive agents, can be detected using medical imaging techniques such as nuclear medicine scans and magnetic resonance imaging (MRI). Imaging agents for magnetic resonance imaging (MRI) include Gd(DOTA), iron oxide or gold nanoparticles; imaging agents for nuclear medicine include 201 Tl, gamma- emitting radionuclide 99 mTc; imaging agents for positron-emission tomography (PET) include positron-emitting isotopes, (18)F-fluorodeoxyglucose ((18)FDG), (18)F-fluoride, copper-64, gadoamide, and radioisotopes of Pb(ll) such as 203Pb, and I lln; imaging agents for in vivo fluorescence imaging such as fluorescent dyes or dye-conjugated nanoparticles. In some embodiments, an agent to be delivered is conjugated, or fused to, or mixed or combined with an imaging agent.

In a further aspect, the invention relates to a pharmaceutical composition comprising the nucleic acid nanostructure of any one of the preceding embodiments and optionally a pharmaceuticals acceptable excipient such as a carrier or a buffer or a salt. A pharmaceutically acceptable carrier facilitates administration of the nanostructures to a cell or a subject such as a human or an animal e.g., a mouse. In one embodiment, the pharmaceutically acceptable excipient is a physiological buffer comprising about 5 mM magnesium (Mg²⁺) salt such as about 0.1 mM to 3 mM, or 0.5 mM to 1 mM Mg²⁺ (e.g., about 0.1 mM, about 0.2 mM, about 0.3 mM, about 0.4 mM, about 0.5 mM, about 0.6 mM, about 0.7 mM, about 0.8 mM or about 0.9 mM Mg²⁺). In some embodiments, the pharmaceutically acceptable excipient is a physiological buffer comprising about 0.5 mM to 1.5 mM calcium (Ca²⁺). For example, the pharmaceutically acceptable excipient is a physiological buffer comprising about 0.5 mM, about 0.6 mM, about 0.7 mM, about 0.8 mM, about 0.9 mM, about 1.0 mM, about 1.1 mM, about 1.2 mM, about 1.3 mM, about 1.4 mM or about 1.5 mM Ca²⁺. In some embodiments, the pharmaceutically acceptable excipient is a physiological buffer comprising about 0.1 mM to 1.5 mM sodium (Na⁺). For example, the pharmaceutically acceptable excipient is a physiological buffer comprising about 0.5 mM, about 0.6 mM, about 0.7 mM, about 0.8 mM, about 0.9 mM, about 1.0 mM, about 1.1 mM, about 1.2 mM, about 1.3 mM, about 1.4 mM or about 1.5 mM Na⁺.

Nucleic acid nanostructures or pharmaceutical compositions of the invention, when delivered systemically, may be formulated for parenteral administration by injection, e.g., by bolus injection or continuous infusion, by intravenous (i.v.) or subcutaneous (s.c.) injection. Formulations for injection may be presented in unit dosage form, e.g., in ampoules or in multidose containers. Pharmaceutical parenteral formulations include aqueous solutions of the ingredients.

The pharmaceutical composition of the invention, wherein the pharmaceutical composition is non-toxic.

Nucleic acid nanostructures of the present invention (including compositions comprising the nanostructures) may be used in a variety of applications, including biomedical applications such as nanomedicine.

In one embodiment, the pharmaceutical composition of the invention or the or the nucleic acid nanostructure of the invention is for use in therapy or diagnosis.

For example, nucleic acid nanostructures may be used for drug delivery (e.g., targeted drug delivery), immunotherapy, diagnostics and molecular biology. An example of such a nucleic nanostructure is a structure as described in EP22207474 with the title "DNA Origami Encoding for Mammalian Gene Expression and Cotransfection" which is incorporated herein per reference, particularly pages 5-19.

For example, nucleic acid nanostructures may be used for trapping pathogens such as living organisms (e.g. viruses) or pathogenic molecules such a protein-based, nucleic acid-based or chemical-based agents as described herein. An example for nucleic acid nanostructures which can be used for virus entrapping is disclosed in WO 2021/165528 which is incorporated herein by reference.

As a further example, the nucleic acid nanostructures of the invention can be used as a membrane protein surrogate.

In one embodiment, the nucleic acid nanostructure of the invention might be used for *in vitro* administration to cells, e.g., research purposes.

In a further aspect, the invention related to a method of producing a nucleic acid nanostructure, preferably the nucleic acid nanostructure of the invention:
(a) providing one or more self-assembling nucleic acid building blocks, preferably DNA building blocks, and providing DNA molecules comprising nuclease-resistant nucleotides,
   wherein the one or more self-assembling nucleic acid building blocks comprise one or more oligonucleotides at pre-defined sites of the self-assembling nucleic acid building blocks, preferably at pre-defined sites on a surface of the nucleic acid nanostructure, wherein the one or more oligonucleotides comprise a nucleic acid sequence stretch which is complementary to one or more DNA sequence stretches of the DNA molecules comprising nuclease-resistant nucleotides; and
(b) subjecting the one or more self-assembling nucleic acid building blocks and the DNA molecules comprising nuclease-resistant nucleotides to conditions which allow complementary base pairing.

In one embodiment, the one or more oligonucleotides and the DNA molecules comprising nuclease-resistant nucleotides in step (a) further comprise at least 2 unpaired pyrimidine nucleotides which are capable of forming pyrimidine dimers. In one embodiment, the unpaired pyrimidine nucleotides are thymidines and the pyrimidine dimers are preferably cyclobutane thymidine dimers. In one embodiment, the unpaired pyrimidine nucleotides are located terminally on proximal DNA strands to connect contiguous DNA strands by a half crossover bond or a full crossover bond. Alternatively, the unpaired pyrimidine nucleotides are located in loops preferably loops of 5 thymidines or proximal DNA strands to form interhelical bonds or branches.

In one embodiment, the conditions which allow complementary base pairing are a temperature between about 4 to 65 °C, preferably about 15 to 37 °C, more preferably about 20 to 27 °C, a pH within a range of about 5.0 to 9.0, monovalent ions such as NaCl or KCI in a range of about 10 mM to 150 mM, preferably about 50 mM to 100 mM, and optionally about 1 mM to 5 mM Mg2+, preferably about 2 mM to 4 mM Mg2+.

In one embodiment, the method comprises a further step (c) of irradiation, preferably irradiation with UV, preferably with about 300-365 nm such as about 310 nm.

In a further aspect, the invention relates to the use of nucleic acid molecules as described herein comprising nuclease resistant nucleotides for protecting nucleic acid nanostructures.

The invention is further illustrated by the following embodiments:
1. A nucleic acid nanostructure, preferably a DNA nanostructure comprising one or more self-assembling nucleic acid building block(s), preferably DNA building block(s), wherein the nucleic acid nanostructure comprises DNA molecules comprising nuclease-resistant nucleotides on at least a part of a surface of the nucleic acid nanostructure.
2. The nucleic acid nanostructure of embodiment 1, wherein the nuclease-resistant nucleotides comprise a modification in the sugar moiety and/or the phosphate moiety, in particular a bridged sugar moiety, an L-sugar moiety, a peptide nucleic acid, and/or a phosphorothioate.
3. The nucleic acid nanostructure of embodiment 1or 2, wherein the nucleic acid nanostructure unfolds and/or decomposes in a time-controlled manner, preferably wherein unfolding is controlled by spatial distribution of the DNA molecules comprising nuclease-resistant nucleotides on the surface of the nanostructure.
4. The nucleic acid nanostructure of any one of the preceding embodiments, wherein the self-assembling nucleic acid building block(s), preferably DNA building block(s), comprise one or more oligonucleotide(s), wherein the one or more oligonucleotide(s) comprise one or more nucleic acid sequence stretch(es) which are complementary to one or more DNA sequence stretch(es) on the DNA molecules comprising nuclease-resistant nucleotides, wherein preferably the DNA molecules comprising nuclease-resistant nucleotides are linked to the self-assembling building block(s) by complementary interactions.
5. The nucleic acid nanostructure of embodiment 4, wherein the DNA molecules comprising nuclease-resistant nucleotides are linked to the self-assembling building block(s) by complementary interactions.
6. The nucleic acid nanostructure of any one of the preceding embodiments, wherein the DNA molecules comprising nuclease-resistant nucleotides are covalently linked to the self-assembling building block(s), in particular by cyclobutane pyrimidine dimers (CPDs).
7. The nucleic acid nanostructure of any of the preceding embodiments, wherein the self-assembling building block comprises:
   i) a single-stranded DNA template, and
   ii) one or more oligonucleotides complementary to said single-stranded DNA template,
   wherein each of said oligonucleotides is either complementary to one contiguous DNA sequence stretch or to at least two non-contiguous DNA sequence stretches on said single-stranded DNA template.
8. The nucleic acid nanostructure of any one of the preceding embodiments, wherein the nucleic acid nanostructure is a shell, capsule, sphere, hemi-sphere, cube, cylinder, a spherocylinder, cone, cuboidal, tube, square, lattice, square lattice, a polyhedron, in particular prism, pyramid, a tetrahedron, octahedron, icosahedron, dodecahedron.
9. The nucleic acid nanostructure of any one of the preceding embodiments, wherein the nucleic acid nanostructure is a shell with an opening for assessing the shell.
10. The nucleic acid nanostructure of any one of the preceding embodiments, wherein the DNA molecules comprising the nuclease-resistant nucleotides comprise about 20 % (m/m), or about 30 % (m/m), or about 40 % (m/m), or about 50 % (m/m), or about 60 % (m/m), or about 70 % (m/m), or about 80 % (m/m), or about 90 % (m/m), or about 100 % (m/m) nuclease-resistant nucleotides relative to the total amount of nucleotides of the DNA molecule.
11. The nucleic acid nanostructure of any one of embodiments 1-10, wherein the DNA molecules comprising the nuclease-resistant nucleotides comprise one or more units comprising the nuclease-resistant nucleotides and one or more DNA units consisting of unmodified nucleotides.
12. The nucleic acid nanostructure of embodiment 11, wherein the one or more units comprising nuclease-resistant nucleotides comprise about 20 % (m/m), or about 30 % (m/m), or about 40 % (m/m), or about 50 % (m/m), or about 60 % (m/m), or about 70 % (m/m), or about 80 % (m/m), or about 90 % (m/m), or about 100 % (m/m) nuclease-resistant nucleotides relative to the total amount of nucleotides of the unit.
13. The nucleic acid nanostructure of embodiment 11 or 12, wherein the one or more units comprising the nuclease-resistant nucleotides and the one or more DNA units consisting of unmodified nucleotides are block copolymers, preferably linear or branched block copolymers.
14. The nucleic acid nanostructure of any one of the preceding embodiments, wherein the self-assembling building blocks comprises or consists of unmodified nucleotides.
15. The nucleic acid nanostructure of any one of the preceding embodiments, wherein the DNA molecules comprising nuclease-resistant nucleotides are single-stranded or double-stranded
16. The nucleic acid nanostructure of any one of the preceding embodiments, wherein the nucleic acid nanostructure, in particular the self-assembling nucleic acid building blocks, further comprises covalent linkages, in particular cyclobutane pyrimidine dimers (CPDs).
17. A pharmaceutical composition comprising the nucleic acid nanostructure of any one of the preceding embodiments and optionally a pharmaceuticals acceptable excipient.
18. The pharmaceutical composition of embodiment 17 comprising the nucleic acid nanostructure linked to an active pharmaceutical ingredient or a diagnostic molecule.
19. The pharmaceutical composition of embodiment 17 or 18, wherein the pharmaceutical composition is non-toxic.
20. The pharmaceutical composition of any one of embodiments 17-19 or the nucleic acid nanostructure of any one of embodiments 1-16 for use in therapy or diagnosis.
21. The pharmaceutical composition of any one of embodiments 17-20 or the nucleic acid nanostructure of any one of embodiments 1-16 for use in targeted drug delivery or in trapping.
22. The pharmaceutical composition of any one of embodiments 17-20 or the nucleic acid nanostructure of any one of embodiments 1-16 for use as a membrane protein surrogate.
23. The pharmaceutical composition of any one of embodiments 17-20 or the nucleic acid nanostructure of any one of embodiments 1-16 for use as in any one of embodiments 20-22, wherein the use comprises i.v. or s.c. administration.
24. Use of the nanostructure of any one of embodiments 1-16 for the *in vitro* administration to cells.
25. A method of producing a nucleic acid nanostructure, preferably the nucleic acid nanostructure of any one of embodiments 1-16:
   (a) providing one or more self-assembling nucleic acid building blocks, preferably DNA building blocks, and providing DNA molecules comprising nuclease-resistant nucleotides, wherein the one or more self-assembling nucleic acid building block(s) comprise one or more oligonucleotide(s) at pre-defined sites of the self-assembling nucleic building block(s), preferably at pre-defined sites on a surface of the nucleic acid nanostructure, wherein the one or more oligonucleotide(s) comprise a nucleic acid sequence stretch which is complementary to one or more DNA sequence stretches of the DNA molecules comprising nuclease-resistant nucleotides; and
   (b) subjecting the one or more self-assembling nucleic acid building block(s) and the DNA molecules comprising nuclease-resistant nucleotides to conditions which allow complementary base pairing.
25. The method of embodiment 24, wherein the one or more oligonucleotide(s) and the DNA molecules comprising nuclease-resistant nucleotides in step (a) further comprise at least 2 unpaired pyrimidine nucleotides which are capable of forming pyrimidine dimers, preferably cyclobutane thymidine dimers, and wherein the method comprises a further step (c) of irradiation, preferably irradiation with UV.
26. Use of DNA molecules comprising nuclease resistant nucleotides for protecting nucleic acid nanostructures.

### Example 1

The following examples illustrate the concept of density of nuclease-resistant nucleotides in double stranded DNA molecules, where strand 1 and strand 2 are each meant to be sequence-complementary, so that a DNA double helix can be formed from the strands 1 and 2. The strands can also be interpreted as sequence segments located within or at the termini of other strands.
Example 1A: Standard DNA with unmodified nucleotides (fast degradation by nucleases possible)
   Strand 1: UUUUUUUUUUUUUUUU
   Strand 2: UUUUUUUUUUUUUUUU
   Example 1B: Completely stabilized (maximum delayed degradation)
   Strand 1: XXXXXXXXXXXXXX
   Strand 2: XXXXXXXXXXXXXX
Example 1C: One strand completely stabilized
   Strand 1: UUUUUUUUUUUUUUU
   Strand 2: XXXXXXXXXXXXXXXXX
Example 1D: One strand partially stabilized
   Strand 1: UUUUUUUUUUUUUUU
   Strand 2: UUXUUXUUXUUXUUXX
      or
   Strand 1: UUUUUUUUUUUUUUU
   Strand 2: UXUXUXUXUXUXUXU
Example 1F: Strands partially cross-stabilized
   Strand 1: UXUXUXUXUXUXUX
   Strand 2: XUXUXUUXUXUXUXU
   Wherein U = unmodified, standard nucleotide; X = nuclease-resistant nucleotide, e.g., LNA.

These different solutions can be used individually or in a combination to control and hinder the activity of certain nucleases, depending on the target specifications.

### Example 2

Figure 6A shows a schematic illustration of a nuclease degradation protection assay. Step 1: Hybridization of handles to the origami structure; Step 2: Nuclease DNase I is added to the origami mixture at 0.002 U/µl; Step 3: Aliquot origami-DNase I mixture and incubate for respective time points at 37 °C; Step 4: After each respective time points, freeze sample at -20 °C and run in an agarose gel.

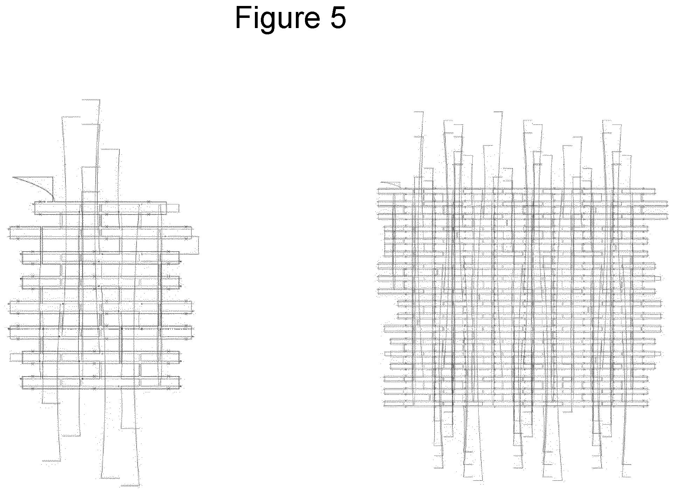

Figure 6B shows a DNase I degradation analysis of s16hb (see Figure 5) with the LNA handle coating density. Laser scanned 2% agarose gels, run at 90V for 2h. NEB 1kB ladder; 0 - 48: Time in hours after adding DNase !. s16hb with DNA handle coating (left) and with LNA handle coating (right). The band indicating unstabilized objects rapidly vanishes within 2-4 hours of exposure to DNase I, whereas the LNA handle coated object persists for up to one day.

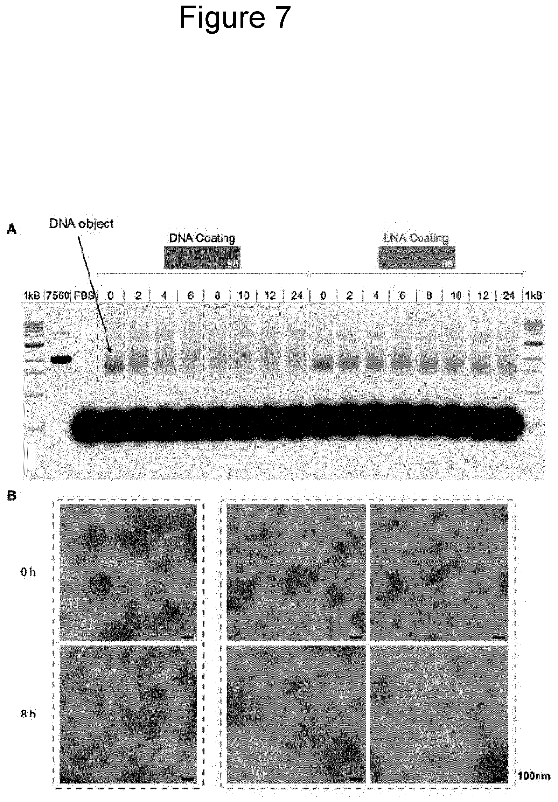

Figure 7A shows the gel electrophoretic mobility of b36hb (see Figure 5). Laser scanned 2% agarose gel, run at 90V for 2h. NEB 1kB ladder; 7560 base scaffold control; FBS control; 0 - 24h: Origami degradation over 24 h. 55 % Fetal Bovine Serum (FBS) was added to DNA coated b36hb (left) and LNA coated b36hb (right). Note that the band patterns are blurry and smeared out due to the additional FBS material in each sample.

Figure 7B shows a negative stain TEM characterization of the degradation of b36hb in 55 % FBS. Negative stain TEM images of 0 h and 8 h time points. In contrast to the LNA coated b36hb, the unmodified DNA coated control has been degraded and intact structures could no longer be observed at 8 h.

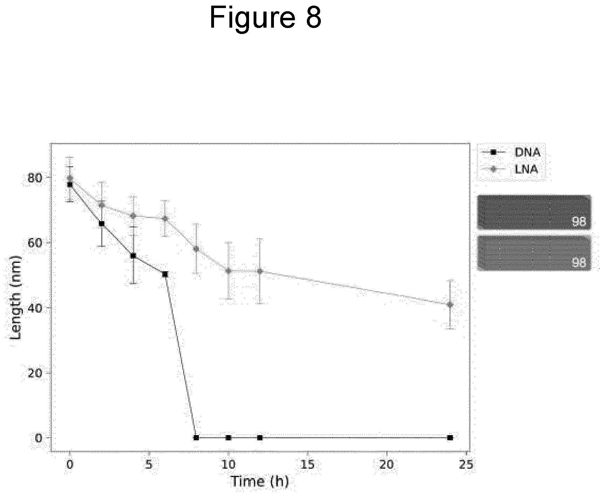

Figure 8 shows the length measurement of the fully coated b36hb throughout 24 h of the DNase I degradation. The length of individual b36hb DNA objects exposed to DNAse I were measured in negative-stain TEM images to illustrate the time dependence of the degradation process. The unmodified standard DNA coated b36hb (black) sample objects rapidly shrunk in size and vanished. Half of the initial length was reached at ~ 7h. The LNA coated b36hb DNA object variant also shrunk but in a much slower fashion. At the endpoint of the experiment at 24hours the particles still had half of their initial length. All scale bars: 100 nm.

## Claims

1. A nucleic acid nanostructure, preferably a deoxyribonucleic acid (DNA) nanostructure, comprising one or more self-assembling nucleic acid building block(s), preferably DNA building block(s), wherein the nucleic acid nanostructure comprises DNA molecules comprising nuclease-resistant nucleotides on at least a part of a surface of the nucleic acid nanostructure.

2. The nucleic acid nanostructure of claim 1, wherein the nuclease-resistant nucleotides comprise a modification in the sugar moiety and/or the phosphate moiety, in particular a bridged sugar moiety, an L-sugar moiety, a peptide nucleic acid, and/or a phosphorothioate.

3. The nucleic acid nanostructure of claim 1 or 2, wherein the nucleic acid nanostructure unfolds and/or decomposes in a time-controlled manner, preferably wherein unfolding is controlled by spatial distribution of the DNA molecules comprising nuclease-resistant nucleotides on the surface of the nanostructure.

4. The nucleic acid nanostructure of any one of the preceding claims, wherein the self-assembling nucleic acid building blocks comprise one or more oligonucleotides, wherein the one or more oligonucleotides comprise one or more nucleic acid sequence stretches which are complementary to one or more DNA sequence stretches on the DNA molecules comprising nuclease-resistant nucleotides.

5. The nucleic acid nanostructure of any one of the preceding claims, wherein the DNA molecules comprising nuclease-resistant nucleotides are covalently linked to the self-assembling building block(s), in particular by cyclobutane pyrimidine dimers (CPDs).

6. The nucleic acid nanostructure of any one of the preceding claims, wherein the DNA molecules comprising the nuclease-resistant nucleotides comprise about 20 % (m/m), or about 25 % (m/m), or about 30 % (m/m), or about 40 % (m/m), or about 50 % (m/m), or about 60 % (m/m), or about 70 % (m/m), or about 80 % (m/m), or about 90 % (m/m), or about 100 % (m/m) nuclease-resistant nucleotides relative to the total amount of nucleotides of the DNA molecule.

7. The nucleic acid nanostructure of any one of the preceding claims, wherein the DNA molecules comprising the nuclease-resistant nucleotides comprise one or more units comprising the nuclease-resistant nucleotides and one or more DNA units consisting of unmodified nucleotides, wherein preferably the one or more units comprising the nuclease-resistant nucleotides and the one or more DNA units consisting of unmodified nucleotides are block copolymers, preferably linear or branched block copolymers.

8. The nucleic acid nanostructure of any one of the preceding claims, wherein the self-assembling building block(s) comprises:
i) a single-stranded DNA template, and
ii) one or more oligonucleotides complementary to said single-stranded DNA template, wherein each of said oligonucleotides is complementary to one contiguous DNA sequence stretch or to two or more non-contiguous DNA sequence stretches on said single-stranded DNA template.

9. A pharmaceutical composition comprising the nucleic acid nanostructure of any one of the preceding claims and optionally a pharmaceuticals acceptable excipient.

10. The pharmaceutical composition of claim 9 comprising the nucleic acid nanostructure linked to an active pharmaceutical ingredient or a diagnostic molecule.

11. The pharmaceutical composition of claim 9 or 10, wherein the pharmaceutical composition is non-toxic.

12. The pharmaceutical composition of any one of claims 9-11 or the nucleic acid nanostructure of any one of claims 1-8 for use in therapy or diagnosis.

13. The pharmaceutical composition of any one of claims 9-11 or the nucleic acid nanostructure of any one of claims 1-8 for use in drug delivery or entrapping, preferably entrapping of pathogenic molecules, viruses and/or organisms.

14. A method of producing a nucleic acid nanostructure, preferably the nucleic acid nanostructure of any one of claims 1-8, the method comprising:
(a) providing one or more self-assembling nucleic acid building block(s), preferably DNA building block(s), and providing DNA molecules comprising nuclease-resistant nucleotides,
wherein the one or more self-assembling nucleic acid building block(s) comprise one or more oligonucleotides at pre-defined sites of the self-assembling nucleic acid building blocks, preferably at pre-defined sites on a surface of the nucleic acid nanostructure, wherein the one or more oligonucleotides comprise a nucleic acid sequence stretch which is complementary to one or more DNA sequence stretches of the DNA molecules comprising nuclease-resistant nucleotides; and
(b) subjecting the one or more self-assembling nucleic acid building block(s) and the DNA molecules comprising nuclease-resistant nucleotides to conditions which allow complementary base pairing.

15. Use of DNA molecules comprising nuclease resistant nucleotides for protecting nucleic acid nanostructures.
